# EUROPEAN PATENT SPECIFICATION

(11) **EP 1 839 703 B1**
(45) Date of publication and mention of the grant of the patent: **25.05.2011**
(21) Application number: 07006065.2
(22) Date of filing: 23.03.2007
(51) Int. Cl.: H01J 65/04

(54) **Phototherapy device**
Phototherapievorrichtung
Dispositif de photothérapie

(30) Priority: 31.03.2006 JP 2006096971
(43) Date of publication of application: 03.10.2007
(73) Proprietor: USHIODENKI KABUSHIKI KAISHA, Chiyoda-ku, Tokyo 100 (JP)
(72) Inventor: Morita, Akimichi, Nagoya-shi, Aichi-ken (JP); Hiramoto, Tatsumi, Tokyo (JP); Sumitomo, Taku, Himeji-shi, Hyogo-ken (JP); Kimura, Makoto, Tokyo (JP); Saga, Takashi, Tokyo (JP)
(74) Representative: Tomerius, Isabel

(56) References cited:
- EP-A1- 0 254 111
- EP-A1- 0 363 832
- WO-A-02/055149
- WO-A-03/024526
- WO-A1-99/30353
- WO-A2-2004/110932
- DE-A1- 10 162 147
- FR-A1- 2 936 093
- US-A- 4 177 384
- US-A- 4 558 700
- US-A1- 2004 147 986
- NOVAK ZOLTAN ET AL: "Efficacy of different UV-emitting light sources in the induction of T-cell apoptosis" PHOTOCHEMISTRY AND PHOTOBIOLOGY, vol. 79, no. 5, May 2004 (2004-05), pages 434-439, XP002440820 ISSN: 0031-8655

## Description

### BACKGROUND OF THE INVENTION

### Field of the Invention

The invention relates to a phototherapy device. The invention relates especially to a phototherapy device for treatment of skin disorders and the like by irradiation with therapeutic radiation which has a spectrum in the wavelength range of UV-B radiation.

### Background of the Invention

In allergic skin disorders, there are various conditions such as, for example, psoriasis, atopic dermatitis, vitiligo and the like. The mechanism, for example, of psoriasis has still not been clarified. Some treatment methods for it have already been suggested, and they are also practiced. However, no processes for completely curing diseased sites of all patients have been found. Only therapies for suppressing the outbreak of the disorder, specifically topical remedies, oral remedies, phototherapy and the like are practiced.

In topical remedies, generally, steroid medicine for topical application, vitamin D₃ medicine for topical application, and the like are used. In steroid medicine for topical application however dermal atrophies and the like occur due to use over a long time. In the vitamin D₃ medicines for topical application, there is the disadvantage that the daily frequency of their use must be limited, and similar disadvantages.

Furthermore, in oral remedies, oral remedies such as retinoid, cyclosporine, and the like are used. However, since they weaken the immune response and suppress abnormal proliferation of the skin, they have the disadvantage that they act effectively not only on diseased sites, but also inevitably on sites outside of diseased sites. Therefore, use for treatment of patients with especially seriously diseased sites is difficult.

On the other hand, phototherapy is a treatment in which, by irradiating white blood cells and T cells, such a lymphoma and the like, which overreact and thus cause allergosis, with light radiation and thus by destruction thereof, allergosis is diminished. It includes a process using light radiation in the UV-A range (320 nm to 400 nm) which is called UV-A radiation and a process using light radiation in the UV-B range (280 nm to 320 nm) which is called UV-B radiation. The treatment process using UV-A radiation is generally called PUVA therapy, it being carried out together with a photosensitive pharmaceutical called psoralen, for example, in that, after oral application or topical application of psoralen or after bathing in a psoralen-containing liquid, irradiation with UV-A radiation is performed. Therefore, in PUVA therapy, unpleasantness occurs in everyday life, such as no sun exposure after this therapy, and the like.

The treatment process using UV-B radiation is, on the other hand, a simple process in which treatment can be administered without using psoralen.
The light source in this UV-B therapy is a fluorescent lamp which contains a phosphor including gadolinium as the activating agent which is put into the discharge space. As a result, radiation is obtained which has a sharp spectrum in the wavelength range from 311 nm to 313 nm (see, for example, Japanese Patent Application JP-A-2004-350946 and corresponding U.S. Patent Application Publication 2005/010249). Another device of this type is disclosed in DE 101 62 147 A1.

Since it can be presumed that a high effect of destruction of white blood cells is obtained by radiation which contains a large proportion of short wavelengths with high energy, there is a demand for a fluorescent lamp which emits radiation which has a spectrum at still shorter wavelengths than in the above described wavelength range. However, since there is no phosphor which intensively emits radiation with this spectrum, it is in fact such UV-B therapy using a fluorescent lamp with a spectrum in the above described wavelength range is carried out.

In view of this circumstance, a XeCl excimer lamp which emits a XeCl excimer light beam has recently been proposed as the light source which is used for the UV-B therapy, for example in DE 105 43 342 A1 and in WO 03/047682 A2. The XeCl excimer radiation, as shown, for example, in Figure 5, has a single emission peak at a wavelength of 308 nm. Since it delivers molecular emission and not an atomic emission, it has a broad spectrum with a wide width. It has the feature that radiation in the short wavelength range of less than or equal to 300 nm is emitted; this is never obtained in a conventionally used fluorescent lamp (see, for example, PCT Patent Application Publication WO 03/024526 and corresponding U.S. Patent Application Publication 2004/0249369 A1, and U.S. Patent 5,955,840).

It is believed that this radiation with a spectrum in the wavelength range including the range with shorter wavelengths of less than or equal to 300 nm is effective for therapy, since a high effect of destruction of T cells, i.e., a high suppression effect of allergosis, is obtained.

The XeCl excimer lamp also has the following advantages:

Since, in a fluorescent lamp, a certain emission is acquired from the phosphor activated by the UV radiation from mercury which has been heated and vaporized during operation by the discharge, the emission intensity changes even by a few dozen %, depending on the amount of vaporization of the mercury within a few minutes after starting of lamp operation. While in UV-B therapy each duration is a few minutes to roughly 10 minutes, control of the amount of irradiation by the duration of irradiation of the diseased sites is difficult. However, in a XeCl excimer lamp, since the emissive material is gaseous even before operation, the emission intensity changes only slightly from the start of lamp operation, by which the amount of irradiation of the diseased sites can be easily controlled.

Furthermore, an XeCl excimer lamp can be operated with a higher power density than a fluorescent lamp, and the diseased sites can be irradiated with therapeutic radiation with high illuminance. This means that irradiation with a constant amount of irradiation in a short time is possible so that shortening of the therapy duration is achieved, yielding the advantage that the burden on the patient can be reduced.

The proportion of short wavelengths in UV-B radiation, on the one hand, yields a high suppression effect of allergosis while, on the other hand, a side effect, specifically DNA damage, is often induced. If damaged DNA is adequately repaired, there is no disadvantage. However, in the worst case, when repair is incomplete, cancer results; but, there is still no proof by numerical values of the correlation between this side effect and the suppression effect of allergosis. Therefore, confirmation of safety in use of such a XeCl lamp is required.

### Summary of the Invention

A primary object of the invention is to devise a phototherapy device that allows for an advantageous use of the device.

The object is achieved in accordance with the invention by a phototherapy device for irradiation of diseased sites as claimed in claim 1.

The object is advantageously achieved in that the phototherapy device in accordance with the invention comprises a radiation emission window in which the light source radiation from the XeCl excimer lamp is incident and via which the therapeutic radiation is emitted.

### Action of the Invention

The phototherapy device in accordance with the invention prevents unwanted side effects by the therapeutic radiation having a specific spectrum with a fixed lower boundary value. Thus T cells in true skin which overreact to allergens can be reduced in the state in which the side effects are suppressed up to a certain degree.

The invention is further described below with reference to the accompanying drawings.

### Brief Description of the Drawings

Figure 1 is a schematic perspective view of the arrangement of one example of the phototherapy device in accordance with the invention;

Figure 2(a) is a schematic front view of the light irradiation unit of the phototherapy device as shown in Figure 1;

Figure 2(b) is a schematic cross-sectional view taken along line A-A in Figure 1;

Figure 2(c) is a schematic cross-sectional view corresponding to that of Figure 2(b) but showing the arrangement in the state in which the frame is open;

Figure 3 is a schematic cross section along the tube axis of one example of a XeCl excimer lamp which is used in the phototherapy device in accordance with the invention;

Figure 4 is a schematic of the arrangement of a power source device for operating the XeCl excimer lamp shown in Figure 3;

Figure 5 is a plot of the spectrum of the light source radiation which is emitted from the XeCl excimer lamp which is used in a phototherapy device;

Figure 6 is a plot of the spectrum of UV-B radiation according to experimental examples;

Figure 7 is an enlarged view of the wavelength range from 290 nm to 305 nm of the spectrum according to Figure 6;

Figure 8 is a plot of the spectrum of the UV-B radiation according to another experimental example;

Figure 9 is a graph representing the apoptosis of white blood cells observed in the experimental examples;

Figure 10 is a graph representing the DNA damage observed in the experimental examples; and

Figure 11 is a graph representing the therapeutic gain in relative terms observed in the experimental examples.

### Detailed Description of the Invention

The phototherapy device in accordance with the invention is described below.

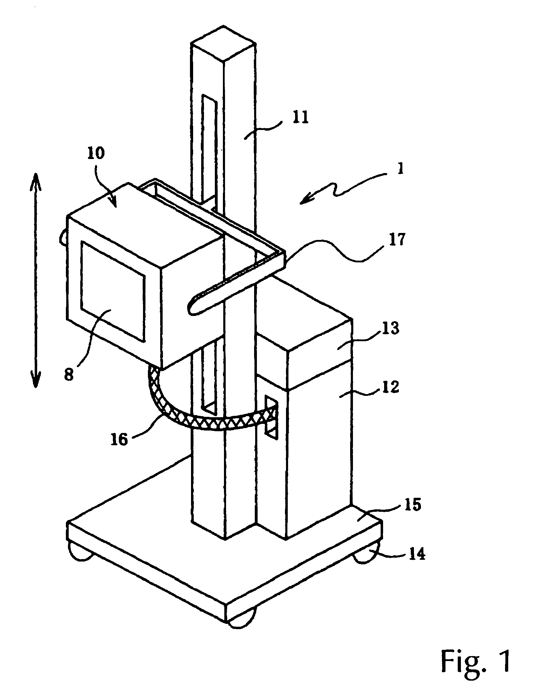

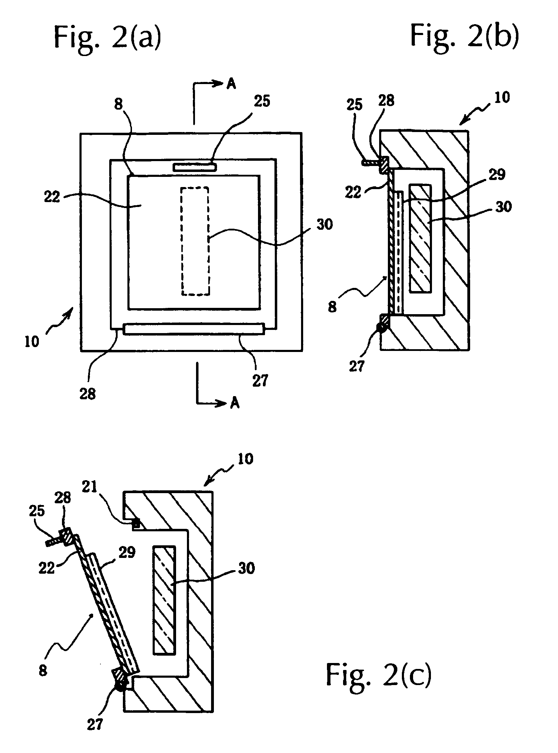

Figure 1 shows a schematic of the arrangement of one example of the phototherapy device in accordance with the invention. Figures 2(a) to 2(c) show the arrangement of one embodiment of the light irradiation unit of the phototherapy device shown in Figure 1. This phototherapy device 1 irradiates diseased sites with therapeutic radiation which has a specific spectrum in the wavelength range of UV-B radiation. The device integrates a light source in the form of a XeCl excimer lamp 30. Furthermore, it has a light irradiation unit 10 which has a radiation emission window 8 via which therapeutic radiation is emitted in one direction. This light irradiation unit 10 is arranged on an arm 17 which can be moved up and down along a vertical stand 11, by which the radiation direction of therapeutic radiation from the radiation emission window 8 can be selected. In the device of Figure 1, there is also a control panel 13, casters 14 which enable movement on a floor, a base frame 15 and a power source part 12 which supplies power by means of the feed line 16 to the light irradiation unit 10.

The radiation emission window 8 of the light irradiation unit 10 in the phototherapy device 1 comprises an optical filter 22 in which the light source radiation from the XeCl excimer lamp 30 is incident and from which therapeutic radiation with a specific spectrum is emitted. This optical filter 22 is held in a slot 29 which is permanently attached in a frame 28. Furthermore, the optical filter 22 is interchangeable, being removable from the slot 29 in that the frame 28 is opened using a hinge mechanism 27 by pulling a handle 25. In Figure 2(c) reference number 21 designates a magnet.

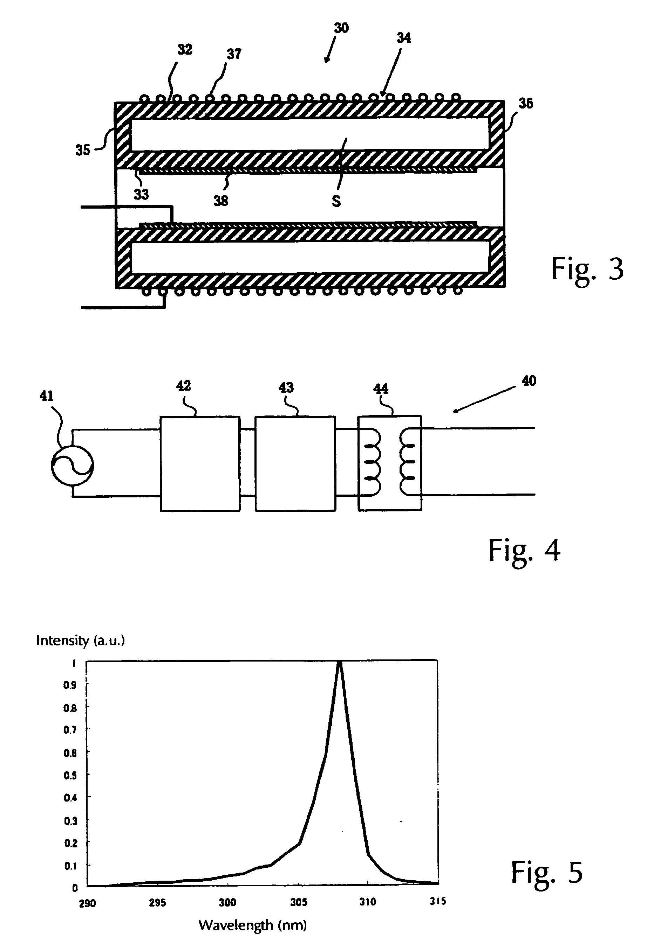

Figure 3 shows the arrangement of an example of a XeCl excimer lamp which is used in the phototherapy device in accordance with the invention in a cross section taken along the tube axis. Figure 4 shows a schematic of the arrangement of a power source device for operating the XeCl excimer lamp of Figure 3.

In the XeCl excimer lamp 30 used in the phototherapy device in accordance with the invention, an excimer molecular emission by xenon gas and chlorine gas is used. The lamp specifically comprises the following:
- a cylindrical outer tube 32 made of a dielectric material, for example, silica glass or the like;
- a cylindrical inner tube 33 made of a dielectric, for example, silica glass or the like, which is coaxially located in the outer tube 32 and which has a smaller outside diameter than the inside diameter of this outer tube 32; and
- a discharge vessel 34 of the hermetically sealed type with a double tube arrangement is formed by the outer tube 32 and the inner tube 33 being hermetically sealed at their ends by end walls 35, 36, so that the discharge vessel 34 encloses a cylindrical discharge space S. The discharge space S is filled with a discharge gas comprised of argon (Ar) gas, xenon (Xe) gas and chlorine (Cl₂) gas.

The outer tube 32 of the discharge vessel 34 is provided with a mesh-type electrode 37 of electrically conductive material such as, for example, a wire net or the like in the state in which the electrode tightly adjoins the outer surface of the outer tube. Moreover, the inner tube 33 is provided with another electrode 38, for example, of an aluminum plate in the state in which it tightly adjoins the inner surface of the inner tube. The electrodes 37, 38 are connected to a power source device 40 which supplies high frequency power to the XeCl lamp 30 to operate it.

The power source device 40 has an AC line power source 41, an AC/DC converter 42, an inverter 43 of semiconductor switching devices, such as, for example, FETS, a transistor and the like, and a transformer 44. The AC/DC converter 42 converts an alternating current (AC) of 100 V which is supplied by the line power source 41 into a direct current, which the inverter 43 converts into a high frequency, which the transformer 44 converts into high voltage of at least some kV, which is necessary to drive the XeCl excimer lamp, and supplies it to the XeCI excimer lamp 30.

The dimensions of the XeCl excimer lamp 30 are described below using one example.
- For the outer tube 32 from which the discharge vessel 34 is formed, the total length is 150 mm, the outside diameter is 26.5 mm and the thickness is 1.0 mm; and
- for the inner tube 33, the total length is 150 mm, the inside diameter is 10.5 mm and the thickness is 1.0 mm.

The discharge vessel 34 is filled with a discharge gas with a pressure of 20 kPa. The sizes of the electrodes 37, 38 are 100 mm in the axial direction of the tube.

Figure 5 shows the spectrum of the light source radiation which is emitted from this XeCl excimer lamp 30.

The therapeutic radiation which is to be emitted onto the diseased sites has a specific spectrum in the wavelength range of UV-B radiation (280 nm to 320 nm) which is continuous, at least in the wavelength range of less than or equal to 303 nm, and moreover, the lower boundary value thereof is in the wavelength range between at least 297 nm and at most 300 nm.

Here, the expression "the spectrum is continuous" means that the emission intensity at each wavelength without interruption is at least 1 %, preferably at least 5 % relative to the peak intensity of the emission peak in the wavelength range of UV-B radiation (280 to 320 nm).

The light source radiation of the XeCI excimer lamp as a light source specifically has an emission peak in the wavelength range from 300 to 315 nm with a spectrum which is continuous at least in a shorter wavelength range from this emission peak up to 295 nm. The therapeutic radiation with a lower boundary value that is controlled to be in the wavelength range from 297 nm to 300 nm is obtained by transmitting the light source radiation through the optical filter 22.

Because the lower boundary value of the spectrum of the therapeutic radiation is in the wavelength range of more than or equal to 297 nm, the DNA damage which forms at diseased sites which have been irradiated with this therapeutic radiation is suppressed at least to a certain degree.

DNA damage can be confirmed by detecting the photoproducts and pyrimidine dimers (6-4) which are produced by this DNA damage with test medicines.

In the above described manner, the optical filter 22 used for this phototherapy device is a means for radiation control which controls the transmission of the light source radiation having short wavelengths in the above described manner and makes it therapeutic radiation with a specific spectrum. This optical filter 22 can be an optical filter of borosilicate glass with silicon dioxide, boric acid and sodium oxide as the main components. The thickness thereof is, for example, 1 mm to 3 mm Furthermore, for example, also water/oxygen-free aluminum fluoride glass can be used in which specifically the contents of BaF₂, CaF₂ and AlF₃ are in the % by mole range from 14.00 to 24.00, in the % by mole range from 28.25 to 38.25 and in the % by mole range from 37.25 to 47.25, respectively, in which, moreover, one of YF₃, SrF₂ and LaF₃ is selected and contained, the content thereof in the case in which YF₃ is contained being 2.5 % by mole to 20 % by mole, it is furthermore in the case in which SrF₂ is contained being 2.5 % by mole to 7.5 % by mole and in the case in which LaF₃ is contained being 2.5 % by mole to 7.5 % by mole, in which moreover Ce is contained and which has a water-free and an oxygen-free composition. Here, it is advantageous that the Ce content is 1 at.% to 10 at.%.

This phototherapy device is operated as follows.

First, the position of the casters 14, the height of the light irradiation unit 10 for a vertical stand 11 and the direction of the arm 17 are adjusted, by which the radiation emission window 8 of the light irradiation unit 10 faces the diseased sites of the patient. Next, for the XeCl excimer lamp 30, when the power source part 12 is turned on the power is supplied via the feed line 16 to the XeCl excimer lamp 30. A given high frequency voltage is applied between the electrodes 37, 38. On the inner surface of the discharge vessel 34 in which the electrodes 37, 38 are located, electrical charges are induced, a strong discharge forms in the space which is highly subject to the electrical field by these induced electric charges, i.e., in the space of the discharge space S which is enclosed by the two electrodes 37 and 38. By this discharge, XeCl excimer radiation as light source radiation is emitted which has a spectrum in the wavelength range of UV-B radiation. Because the light source radiation emitted from this XeCl excimer lamp 30 is transmitted by the optical filter 22, of which the radiation emission window 8 is formed, and transmitted radiation is controlled in short wavelengths, the light source radiation is converted into therapeutic radiation with a specific spectrum and emitted onto the diseased sites.

In the above described phototherapy device, because the therapeutic radiation has a specific spectrum with a controlled lower boundary value, occurrence of unexpected side effects is suppressed, and T-cells in true skin which overreact to allergens can be reduced such that side effects are suppressed at least up to a certain degree.

The phototherapy device in accordance with the invention is not limited to the above described embodiment, but various changes can be undertaken on it.

(1) The means for radiation control is not limited to the optical filter which is located between the light source and the diseased site, but there can be various means in different arrangements.
(2) There is no limitation to an arrangement in which the light source and the means for radiation control for controlling the lower boundary of the spectrum of the phototherapy device are arranged separately from one another, but a one-piece arrangement of the light source with the radiation control means can also be undertaken, in which for example the light source is a XeCl excimer lamp and on the surface of the outer tube of this XeCl excimer lamp a filter film which is formed, for example, of a dielectric multilayer film or the like is formed, or similar arrangements.

Experimental examples of the invention are described below. However, the invention is not limited to these experimental examples.

### (Experimental examples 1 to 5)

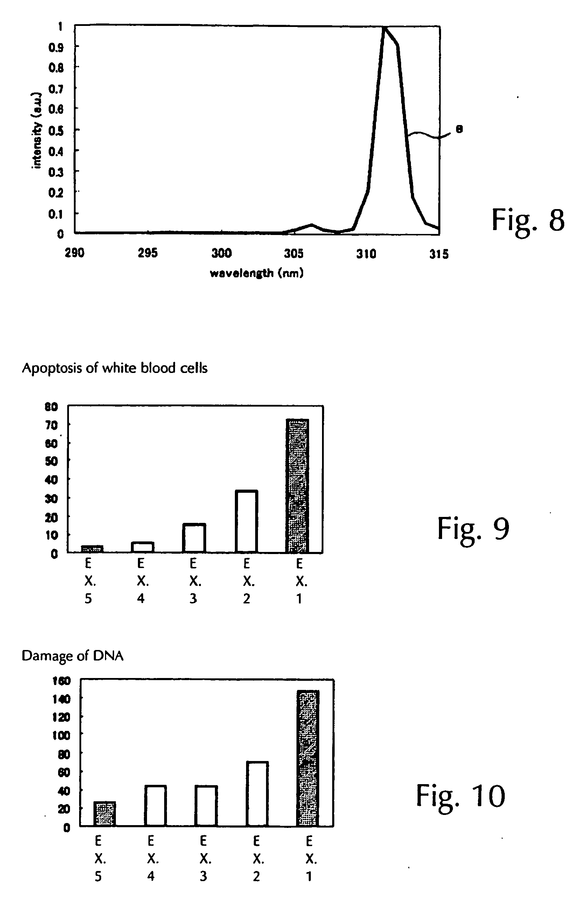

A Petri dish is filled with a phosphate buffer solution (PBS solution) which contains a certain number of white blood cells. Phototherapy samples a to e were irradiated for 50 seconds (further described below) under the conditions that the energy density is roughly 2 mW/cm² and the entire amount of irradiation energy is 100 mJ and then the phototherapy samples a to e were cultured for 24 hours. Afterward, the apoptosis of white blood cells was measured by the conventional fluorine inert process. Figure 9 shows the results.

Furthermore, phototherapy samples a to e which were obtained in the same way were allowed to react with enzyme which reacts on damaged DNA sites. Moreover, this enzyme was allowed to react with a dye and the damaged sites were tagged. DNA damage was measured by the conventional ELIZA process. Figure 10 shows the results, experimental example 1 relating to UV-B radiation a, experimental example 2 relating to UV-B radiation b, experimental example 3 relating to UV-B radiation c, experimental example 4 relating to UV-B radiation d and experimental example 5 relating to UV-B radiation e.

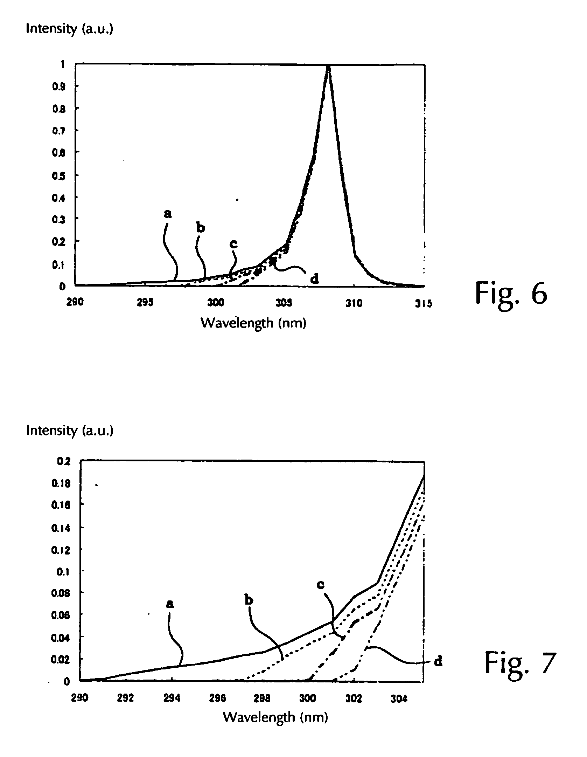

UV-B radiation b to d according to experimental examples 2 to 4 is therapeutic radiation which is obtained by the light source radiation from the XeCl excimer lamp with the arrangement of optical filters which are shown in Figure 1 being transmitted for suppressing the transmission of the proportion of short wavelengths, and which is formed of borosilicate glass with thicknesses of 1.0 mm, 1.5 mm, and 3.0 mm. Figures 6 and 7 show spectra thereof.

UV-B radiation a according to experimental example 1 is radiation of light source radiation of the XeCl excimer lamp in itself with the arrangement shown in Figure 1 without using the optical filter. Figures 6 and 7 show its spectra.

Furthermore, UV-B radiation e according to experimental example 5 is radiation from light source radiation of the fluorescent lamp in itself which is used for conventional UV-B therapies and in which the phosphor added to the discharge space contains gadolinium. Figure 10 shows the spectrum.

As is apparent from Figure 9, the larger the proportion of short wavelengths of the UV-B radiation, the higher the apoptosis of white blood cells. In this connection, it was confirmed that the suppression effect of the allergosis can be adequately obtained. However, as is apparent from Figure 10, it was confirmed that the greater the proportion of short wavelengths of UV-B radiation, the higher the DNA damage as a side effect.

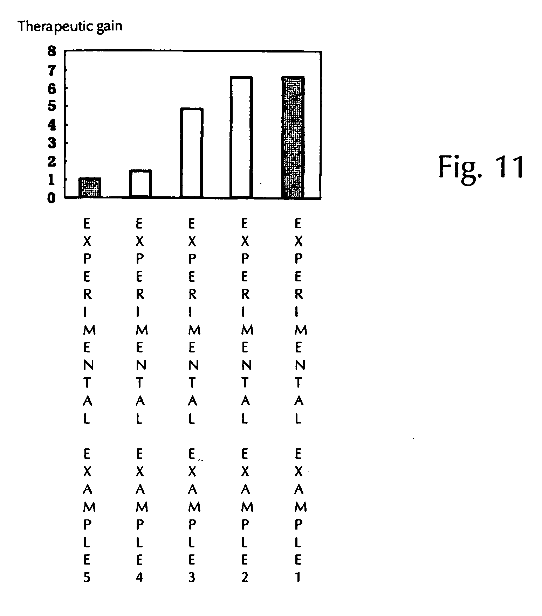

Therefore, the ratio of the apoptosis of the white blood cells to the DNA damage was computed and the amount of therapeutic effect compared to the side effects was represented as the therapeutic gain using numerical values. This therapeutic gain shows that the greater it is, a high therapeutic action relative to side effects is obtained, and that the smaller it is, there is only a small therapeutic effect relative to the side effects. Figure 11 shows the result.

Figures 9 to 11 show the following:

For UV-B radiation a to d according to experimental examples 1 to 4, in which the spectra are continuous in the wavelength range of less than or equal to 303 nm, the apoptosis of the white blood cells is higher, and also the DNA damage as a side effect is higher, the greater the proportion of short wavelengths which the radiation contains, i.e., the smaller the lower boundary value becomes in the short wavelengths. By comparison of the UV-B radiation a, with a lower boundary value in the short wavelengths at a wavelength of 290 nm, with UV-B radiation b with a lower boundary value in the short wavelengths at a wavelength of 297 nm the therapeutic gain of the UV-B radiation a is clearly at most almost equal to that of the UV-B radiation b with a smaller amount of short wavelengths than this. This UV-B radiation a has a small therapeutic effect in spite of large side effects.

In this case of the almost identical therapeutic gain, with consideration of individual differences and the like with respect to the DNA damage it is advantageous to use UV-B radiation with a risk of side effects as small as possible, i.e., with low DNA damage. On the other hand, for the UV-B radiation e according to experimental example 5 in which the lower boundary value of the spectrum is 304 nm and in which it cannot be mentioned that the spectrum is continuous in a wavelength range of less than or equal to 303 nm, it was confirmed that the apoptosis of the white blood cells, DNA damage and therapeutic gain are low.

It can be recognized from the above described circumstance that it is necessary for therapeutic radiation that the spectrum be continuous in a wavelength range of less than or equal to 303 nm, and that moreover, the lower boundary value thereof is in the wavelength range of more than or equal to 297 nm.

Furthermore, it is shown that the lower the lower boundary value, the higher the therapeutic gain becomes when the lower boundary value is in the wavelength range of less than or equal to 303 nm. Specifically, the therapeutic radiation according to experimental example 3 (lower boundary value: 300 nm) is more advantageous than the therapeutic radiation according to experimental example 4 (lower boundary value: 301 nm). Furthermore, it was confirmed that the therapeutic radiation according to experimental example 2 (lower boundary value: 297 nm) is more advantageous than the therapeutic radiations according to experimental examples 3 and 4.

## Claims

1. Phototherapy device (1) for irradiation of diseased sites with therapeutic radiation with a spectrum in the wavelength range of UV-B radiation, comprising means for irradiating diseased sites with a continuous spectrum of therapeutic radiation, wherein the means for irradiating comprises a light source which emits light source radiation wherein the light source comprises a XeCl excimer lamp (30) with a discharge space (5), **characterized in that** the discharge space (5) is filled with a discharge gas comprised of argon gas, xenon gas and chlorine gas, and comprising an optical filter (22) which controls the lower boundary value of the spectrum of the light source radiation such that the spectrum of the therapeutic radiation is continuous in a wavelength range of at most 303 nm and the lower boundary value of this continuous spectrum of the therapeutic radiation is in the wavelength range between at least 297 nm and at most 300 nm,
wherein the optical filter (22) is of borosilicate glass with silicon dioxide, boric acid and sodium oxide as the main components or
of water/oxygen-free aluminum fluoride glass in which the contents of BaF₂, CaF₂ and AlF₃ are in the % by mole range from 14.00 to 24.00, in the % by mole range from 28.25 to 38.25 and in the % by mole range from 37.25 to 47.25, respectively, in which, moreover, one of YF₃, SrF₂ and LaF₃ is selected and contained, the content thereof in the case in which YF₃ is contained being 2.5 % by mole to 20 % by mole, it is furthermore in the case in which SrF₂ is contained being 2.5 % by mole to 7.5 % by mole and in the case in which LaF₃ is contained being 2.5 % by mole to 7.5 % by mole, in which moreover Ce is contained and which has a water-free and an oxygen-free composition.

2. Phototherapy device (1) in accordance with claim 1, which has a radiation emission window (8) in which the light source radiation from the XeCl excimer lamp (30) is incident and via which the therapeutic radiation is emitted.

3. Phototherapy device (1) in accordance with claims 1 or 2, wherein the optical filter (22) forms said radiation emission window (8).

4. Phototherapy device (1) in accordance with claims 1, 2 or 3, wherein the thickness of the optical filter (22) of borosilicate glass is 1 mm to 3 mm.

5. Phototherapy device (1) in accordance with claim 1, 2 or 3, wherein the Ce content of the water/oxygen-free aluminum fluoride glass of the optical filter (22) is 1 at. % to 10 at. %.

## Patentansprüche

1. Phototherapievorrichtung (1) zum Bestrahlen erkrankter Stellen mit einer therapeutischen Strahlung mit einem Spektrum im Wellenlängenbereich der UV-B-Strahlung, umfassend ein Mittel zum Bestrahlen erkrankter Stellen mit einem kontinuierlichen Spektrum therapeutischer Strahlung, wobei das Bestrahlungsmittel eine Lichtquelle umfasst, die eine Lichtquellenstrahlung aussendet, wobei die Lichtquelle eine XeCl-Excimerlampe (30) mit einem Entladungsraum (5) umfasst,
**dadurch gekennzeichnet, dass**
der Entladungsraum (5) mit einem Entladungsgas gefüllt ist, das aus Argongas, Xenongas und Chlorgas gebildet ist, und einen optischen Filter (22) umfasst, der den unteren Grenzwert des Spektrums der Lichtquellenstrahlung derart steuert, dass das Spektrum der therapeutischen Strahlung in einem Wellenlängenbereich von höchstens 303 nm kontinuierlich ist und dass sich der untere Grenzwert dieses kontinuierlichen Spektrums der therapeutischen Strahlung im Wellenlängenbereich zwischen mindestens 297 nm und höchstens 300 nm befindet,
wobei der optische Filter (22) aus Borsilikatglas mit Siliciumdioxid, Borsäure und Natriumoxid als Hauptbestandteilen besteht oder
aus wasser-/sauerstofffreiem Aluminiumfluoridglas, wobei sich der Gehalt an BaF₂, CaF₂ und AlF₃ im Bereich von 14,00 bis 24,00 Mol-%, im Bereich von 28,25 bis 38,25 Mol-% bzw. im Bereich von 37,25 bis 47,25 Mol-% bewegt, wobei darüber hinaus eines aus YF₃, SrF₂ und LaF₃ ausgewählt wird und enthalten ist, wobei der Gehalt im Fall, dass YF₃ enthalten ist, 2,5 Mol-% bis 20 Mol-% beträgt, wobei ferner der Gehalt im Fall, dass SrF₂ enthalten ist, 2,5 Mol-% bis 7,5 Mol-% beträgt und im Fall, dass LaF₃ enthalten ist, 2,5 Mol-% bis 7,5 Mol-% beträgt, wobei darüber hinaus Ce enthalten ist und wobei die Zusammensetzung wasserfrei und sauerstofffrei ist.

2. Phototherapievorrichtung (1) nach Anspruch 1,
die ein Strahlungsemissionsfenster (8) aufweist, in welches die Lichtquellenstrahlung von der CeCl-Excimerlampe (30) einfällt und durch welches die therapeutische Strahlung ausgesendet wird.

3. Phototherapievorrichtung (1) nach Anspruch 1 oder 2,
wobei der optische Filter (22) das Strahlungsemissionsfenster (8) bildet.

4. Phototherapievorrichtung (1) nach Anspruch 1, 2 oder 3, wobei die Dicke des optischen Filters (22) aus Borsilikatglas 1 mm bis 3 mm beträgt.

5. Phototherapievorrichtung (1) nach Anspruch 1, 2 oder 3,
wobei der Ce-Gehalt des wasser-/sauerstofffreien Aluminiumfluoridglases des optischen Filters (22) 1 At.-% bis 10 At.-% beträgt.

## Revendications

1. Un dispositif de photothérapie (1) pour l'irradiation de sites malades avec une radiation thérapeutique avec un spectre dans la fourchette de longueur d'ondes de radiation UVB, comprenant un moyen d'irradier des sites malades avec un spectre continu de radiation thérapeutique, où le moyen d'irradier comprend une source de lumière qui émet une radiation de source de lumière où la source de lumière comprend une lampe à excimère XeCl (30) avec un espace de décharge (5),
**caractérisé en ce**
**que** l'espace de décharge (5) est rempli d'un gaz de décharge composé d'argon, de xénon et de gaz chlore, et comprenant un filtre optique (22) qui contrôle la valeur limite inférieure du spectre de radiation de source de lumière de sorte que le spectre de la radiation thérapeutique soit continu dans une fourchette de longueur d'ondes d'au maximum 303 nm et la valeur limite inférieure de ce spectre continu de la radiation thérapeutique soit dans la fourchette de longueur d'ondes entre au moins 297 nm et au plus 300 nm,
où le filtre optique (22) est en verre borosilicaté avec du dioxyde de silicone, de l'acide borique et de l'oxyde de sodium comme composants principaux ou
en verre au fluorure d'aluminium sans oxygène/eau dans lequel les contenus de BaF₂, CaF₂ et AlF₃ sont dans la fourchette de % par mole de 14,00 à 24,00, dans la fourchette de % par mole de 28,25 à 38,25 et dans la fourchette de % par mole de 37,25 à 47,25, respectivement, dans lequel, de plus, l'un des YF₃, SrF₂ et LaF₃ est sélectionné et contenu, le contenu, dans le cas où YF₃ serait contenu, étant de 2,5% par mole à 20% par mole, cela étant, de plus, dans le cas où SrF₂ serait contenu, de 2,5% par mole à 7,5% par mole et dans le cas où LaF₃ serait contenu, de 2,5% par mole à 7,5% par mole, dans lequel, de plus, Ce est contenu et qui a une composition sans eau et sans oxygène.

2. Un dispositif de photothérapie (1) selon la revendication 1,
qui a une fenêtre d'émission de radiation (8) dans laquelle la radiation de la source de lumière de la lampe à excimère XeCl (30) est incidente et au travers de laquelle la radiation thérapeutique est émise.

3. Un dispositif de photothérapie (1) selon les revendications 1 ou 2,
où le filtre optique (22) forme ladite fenêtre d'émission de radiation (8).

4. Un dispositif de photothérapie (1) selon les revendications 1, 2 ou 3,
où l'épaisseur du filtre optique (22) en verre borosilicaté est de 1 mm à 3 mm.

5. Un dispositif de photothérapie (1) selon la revendication 1, 2 ou 3,
où le contenu en Ce du verre au fluorure d'aluminium sans eau/oxygène du filtre optique (22) est de 1 at.% à 10 at.%.
